# EUROPEAN PATENT SPECIFICATION

(11) **EP 3 727 341 B1**
(45) Date of publication and mention of the grant of the patent: **12.01.2022**
(21) Application number: 18830227.7
(22) Date of filing: 18.12.2018
(51) Int. Cl.: A61K 9/16, A61K 45/06, A61K 9/20, A61K 9/28, A61K 31/155, A61K 31/702

(54) **FIXED DOSE COMBINATION TABLET FORMULATION OF ACARBOSE AND METFORMIN AND PROCESS FOR PRODUCING THE SAME**
TABLETTENFORMULIERUNG AUS ACARBOSE UND METFORMIN MIT FESTGELEGTER DOSIS UND VERFAHREN ZU IHRER HERSTELLUNG
FORMULATION DE COMPRIMÉ COMBINÉE À DOSE FIXE D'ACARBOSE ET DE METFORMINE ET SON PROCÉDÉ DE PRODUCTION

(30) Priority: 18.12.2017 EP 17208069
(43) Date of publication of application: 28.10.2020
(73) Proprietor: Bayer Aktiengesellschaft, 51373 Leverkusen (DE)
(72) Inventor: ANLAHR, Johanna, 44139 Dortmund (DE)
(74) Representative: BIP Patents
(86) International application number: PCT/EP2018/085501
(87) International publication number: WO 2019/121685

(56) References cited:
- WO-A1-2004/110422
- WO-A1-2007/131930
- WO-A1-2013/115742
- WO-A2-2009/135951
- DE-A1- 19 860 698
- US-A1- 2006 222 709
- US-A1- 2014 287 040

## Description

The present invention is related to a tablet formulation comprising the glycosidase inhibitor acarbose (O-4,6-Didesoxy-4-{[(1S,4R,5S,6S)-4,5,6-trihydroxy-3-(hydroxymethyl)-2-cyclohexen-1-yl]amino}-α-D-glucopyranosyl-(1,4)-O-α-D-glucopyranosyl-(1,4)-D-glucopyranose) and the glycerin-3-phosphate-Dehydrogenase inhibitor metformin (1,1-Dimethylbiguanid), particularly the hydrochloride salt of metformin.

Acarbose has been first brought to the market by Bayer in the early 1980's under the still used and sold brand name Glucobay^{®} and different formulations of metformin are on sale for more than 60 years as well, for instance under the brand name Glucophage^{®}.

In the meantime there has been extensive research and development directed to improved formulations for either acarbose or metformin.

Just recently it has been found that a fixed dose combination of acarbose and metformin at 500 mg acarbose combined with 50 mg metformin (which was used in its salt form of metformin-hydrochloride) displays surprisingly superior antihyperglycaemic efficacy when compared to a acarbose monotherapy (J.S. Wang et al., Diabetes Res. Clin Pract (2013), 102(1), 16-24).

Given the fact that many people suffer from type 2 diabetes or are at risk to develop that disease a more effective treatment option, such as the above referred to fixed dose combination are eagerly sought, particularly for societies which have - due to dietary and genetically derived metabolic differences - a dramatically increasing prevalence of type 2 diabetes. Furthermore the overall incidence rate of Type 2 diabetes is increasing with the average age of a population.

In result thereto, particularly for China there is a need to have such fixed dose combination available, as all of the above referred to effects combine. Due to the rapid economic development of China, overall age (e.g. life expectancy and average age) of Chinese people is increasing dramatically alongside with changing dietary behaviors and a metabolic predisposition that both increase the risk of type 2 diabetes.

From a tablet formulation perspective, such circumstances impose further restrictions and hurdles on the development of a fixed dose combination tablet.

While elderly people have in general difficulties to swallow bigger tablets, the Asian population including the Chinese society have additional physiological difficulties in swallowing those (A. Mohaptra et al., "Formulation, development and evaluation of patient friendly dosage forms of metformin, Part-1: Orally disintegrating tablets.", Asian Journal of Pharmaceutics, 07-09 2008).

From that, it results that a tablet formulation that is later supposed to form the basis for a tablet for Asian patients should be as small as possible which translates into the overall tablet weight being as low as feasible at a given drug load. Accordingly, any and all excipients added to the tablet formulation in addition to the active substance(s) should be kept as minute as possible to facilitate a small tablet. For example, the number of excipients, the amount of each excipient, and the total amount of excipients should be as low as possible.

There are many tablet formats available and known. It has been found that amongst those, tablets of not more than 18 mm length, a width of not more than 8 mm and a thickness of not more than 7 mm are those that are well accepted on the Asian market as of being comparably easy to swallow while still allowing - due to their size - to reasonably formulate a sufficient amount of active in that format.

In the context of the present invention, pertaining to a fixed dose combination of acarbose and metformin suitable to be formulated in small tablets for the Asian market, the above requirements meet two additional boundary conditions that make identifying a fixed dose combination formulation of acarbose and metformin particularly difficult.

The first boundary condition is derived from the absolute size of the Chinese population with a relatively high and increasing prevalence of Type 2 Diabetes and the generic nature of both active ingredients. Those two facts require, that any formulation developed for a fixed dose combination must facilitate production of tablets in very high amounts at a prize that is at least in the range of the tablets with the respective single actives. This translates that the formulation must be capable to be handled in an automated very high throughput production process to allow to meet those requirements.

The second boundary condition is derived from the inherent substance matter properties of the two actives to be formulated in a single fixed dose tablet. According to the 9th Edition of European Pharmacopoeia (Ph.Eur.) 2017 (9.2) acarbose is a white or yellowish, hygroscopic, amorphous powder, while metformin (in the form of its HCl salt) is a white or almost white crystal that is well known to exhibit poor compressibility during compaction, often resulting in weak and unacceptable tablets that have a high tendency to cap (B. Barot et al., "Development of directly compressible metformin hydrochloride by the spray-drying technique", Acta Pharm. (2010) 68, 165-175). Likewise to acarbose, metformin is hygroscopic.

For high throughput and very high throughput production processes >10.000, e.g > 40500 and sometimes even > 100.000 tablets are produced per hour. These processes thus pose particular challenges for fixed dose combination formulations comprising metformin and acarbose and tablets thereof. In these processes capping was found to occur as a major issue affecting reproducibility and sustainability of production of metformin/acarbose tablets with optimal stability, such as mechanical stability, disintegration and swallowability of the tablet while maintaining optimal stability of the active ingredients. Capping refers to undesired breaking of the tablet, e.g. when the top (cap) of the tablet splits or fractures from the body of the tablet. Without being bound by theory capping is assumed to be caused by non-compressible fines (small dust particles) that migrate when the air is pushed out during compression. These fines collect at the junction of the upper cup edge and the tablet band.

Thus, such material properties need to be carefully considered when trying to find a suitable tablet formulation of a fixed dose combination of acarbose and metformin. Many attempts have been taken to improve the available formulations of difficult to formulate actives such as metformin or acarbose.

Mostly such attempts pertain to adding new binders addressing the material properties of the actives. Some of the attempts also pertained to new formulation processes as well as particularly to screening for better binders that compensate for the poor properties of metformin.

From the screening of suitable binders, mainly polyvinylpyrollidones (PVPs), cross-linked PVPs (crospovidones), vinylpyrrolidone-vinylacetate copolymers (copovidones), microcrystalline cellulose (MCC), hydroxypropylcellulose (HPC), hydroxypropylmethylcellulose (HPMC, hypromellose) and several grades of polyethylene glycole (PEG, macrogol) have evolved as suitable candidates.

Herting et al. made a "Comparison of different dry binders for roll compaction/dry granulation" (Pharm. Dev. & Techn. 12 (2007) 525-532) exemplifying that use of hydroxypropylcellulose significantly increases disintegration times and thus release of actives from tablets (see Herting et al Figure 6), making hydroxypropylcellulose a preferred candidate for retard formulations.

Mangal et al. ("Roll compaction/Dry granulation: suitability of different binders", Int. Jour. of Pharmaceutics 208 (2016) 213-219) showed that the also difficult to formulate active paracetamol was found to be improved in formulation behavior towards a tablet by adding certain hydroxpropylcellulose as a dry binder, whereas hydroxpropylcellulose was still deemed to be inferior to the use of copovidones, such as the Kollidon^{®} VA64 grade exemplified therein.

In principle, said finding is confirmed also by later work of the same group, wherein Arndt et al. ("Influence of binder properties on dry granules and tablets", Powder Technol. (2017), http://dx.doi.org/10.1016/j.powtec.2017.04.054) state that hydroxypropylcellulose is not to be recommended in cases, where disintegration time of the later tablet is relevant to be optimized. In those instances Kollidon ^{®} VA 64 fine could be chosen.

All of the above referred attempts to use hydroxpropylcellulose as an excipient were made using several actives different to either acarbose or metformin. All of the above referred findings are also supported by publications relating to metformin containing formulations.

With regard to metformin, it is mostly confirmed that copovidone (such as Kollidon^{®} VA64) blended formulations are superior to hydroxpropylcellulose blended formulations. According to Takasaki et al. Kollidon^{®} VA64 exhibited no problems, while hydroxpropylcellulose negatively affected the further handling of the formulation ("The importance of binder moisture content in metformin HCL high-dose formulations prepared by moist aqueous granulation (MAG)", Res. Pharm Sci. 5 (2015) 1-7).

That pyrrolidone based binders are preferred in metformin tablet formulations is also confirmed by L.C. Block et al. in "Pharmaceutical equivalence of metformin tablets with various binders" (Rev. Cienc. Farm. Basica Apl. v 29, (2008) 29-35) comparing several binders, such as different starches and polyvinylpyrrolidones in wet granulation, wherein the polyvinylpyrrolidones were found to be superior.

Within US 6,117,451 hydroxpropylcellulose is used from 0.1 to 15% by weight on a dry weight basis, while such hydroxpropylcellulose has an average molecular weight of 800,000 g/mol to 1,200,000 g/mol and a particle size of about 177 to 590 µm. According to US 6,117,451, said hydroxpropylcellulose is used to form a dynamic hydrophilic matrix system that slows the release of the metformin from the tablet, underlining the retard/slow release function of hydroxpropylcellulose as confirmed before by Herting et al.

In conclusion, the prior art pertaining to tablet formulations of metformin, trying to address the material properties of metformin by addition of suitable binders has identified vinylpyrrolidone based binders to be the best choice, while addition of hydroxpropylcellulose is recommended to be added in case a slow release formulation is intended.

However, none of the above referred to references is concerned with a fast releasing combined formulation of metformin and acarbose and accordingly none thereof addresses the specific challenges of such combination.

That the combination of metformin with another active renders the formulation issues even more severe has been proven by Lakshman et al. ("Application of melt granulation technology to enhance tableting properties of poorly compactible high-dose drugs", Jour. Pharm. Sci. 100 (2011) 1553-1565), who have outlined that excipients with beneficial physicochemical properties must be used to overcome the less desirable properties of drug substances (such as metformin) and that therefore high percentages of excipients are used compared to the amount of actives. Lakshman et al. have furthermore outlined that this contravenes the aim to obtain tablets which are as small as possible for easier swallowing. Lakshman et al. therefore propose to use a different (melt granulation) process of manufacturing to address that issue. In that context Lakshman et al. discloses a formulation comprising metformin, a hydroxypropylcellulose (Klucel^{®} EXF - a high molecular weight hydroxypropylcellulose) and a - not further specified - second drug substance together with magnesium stearate to be used in such melt granulation process.

It is shown that capping is a severe issue if the hydroxypropylcellulose of the reference is not used in sufficient amounts.

Furthermore Lakshman et al. show that addition of hydroxypropylcellulose successively increases the disintegration time of the melt granulated and pressed tablets. From the above, it can be concluded and summarized that up to today no formulation for a fixed dose combination of acarbose and metformin has been described that can overcome the difficulties of providing with a tablet that is easy to swallow, fast to disintegrate and release the actives, while said tablet can reliably be manufactured in a very high throughput production process without suffering from too many tablets that need to be discarded for having capped. Without being bound to a theory on why there is no publication pertaining to a combined acarbose and metformin tablet formulation, this may be explained by the pure fact that the *per se* difficult to formulate metformin needs to be added to acarbose, as the material properties of the two actives are negatively stacking and combination of a crystalline (metformin) and an amorphous (acarbose) agent is inherently problematic, as the formulated and tableted product is a pharmaceutical composition, with particular demands on product uniformity that is hampered by the fact of having to add an amorphous active.

It is therefore an object of the present invention to provide with a fixed dose tablet formulation of metformin and acarbose that allows manufacturing of a small tablet which is easy to swallow and fast to disintegrate and to release the actives, while said tablet can reliably be manufactured in a very high throughput production process without suffering from too many tablets that need to be discarded for having capped. The resulting tablet is therefore also an aspect of the present invention.

It is furthermore an object of the present invention to provide with a fixed dose tablet formulation of metformin and acarbose that allows reliable, stable and sustainable manufacturing of easy-to-swallow tablets in a high throughput or very high throughput production process, wherein the tablets have optimal stability and disintegration behaviour and wherein the active ingredients have an acceptable shelf life during storage. It is another object of the present invention to provide a manufacturing process that is suited to manufacture fixed dose combination tablets out of the formulation according to the present invention that also addresses the material properties of both acarbose and metformin.

It is furthermore an object of the present invention to provide with a high throughput or very high throughput production process to produce such a fixed dose tablet formulation of metformin and acarbose. Therefore such process must not require elevated temperature processing steps nor addition of excess amounts of water, as both actives are hygroscopic and particularly acarbose is comparably sensitive to higher temperatures as well as being subject to potential degradation upon uptake of excess amounts of water.

### Definitions

'Tablets' are a solid pharmaceutical dosage form containing drug substance, e.g. in combination with suitable diluents or excipients and can be prepared e.g. by either compression or molding methods as known in the art. Tablets may or may not be coated as described below.

For the purpose of the present invention, a 'fixed-dose tablet formulation' or 'fixed-dose combination tablet formulation' refers to a formulation comprising two or more active pharmaceutical ingredients. Fixed-dose tablet formulation can be arranged in the form of a single dosage form, such as a tablet. Suitable active ingredients according to the current invention can be metformin and acarbose. According to the current invention the fixed dose may refer to a weight ratio of acarbose to metformin between 1 to 17 and 1 to 4. Preferably, said ratio is about 1 to 12.5, 1 to 10 (e.g. 50 mg acarbose with 500 mg metformin), or about 1 to 8.5 (e.g. 100 mg acarbose with 850 mg metformin) or about 1 to 5 (e.g. 100 mg acarbose with 500 mg metformin). Fixed-dose tablet formulations may comprise further excipients, such as binders, disintegrants, excipients with disintegration promoting properties, sustained-release polymers, lubricants, flow agents, diluents, flavors and colorants or various others.

For the purpose of the present invention, 'metformin' (CAS No. 657-24-9) is to be understood as 1,1-Dimethylbiguanide, as well as any solvate, hydrate or salt thereof. Preferably, metformin is used in its hydrochloric salt form.

For the purpose of the present invention 'acarbose' (INN, CAS No. 56180-94-0) is to be understood as O-4,6-Didesoxy-4-1[(1S,4R,5S,6S)-4,5,6-trihydroxy-3-(hydroxymethyl)-2-cyclohexen-1-yl]amino}-α-D-glucopyranosyl-(1,4)-O-α-D-glucopyranosyl-(1,4)-D-glucopyranose, as well as any solvate, hydrate or salt thereof. Preferably pure acarbose is used.

In general and according to the present invention, 'binders' hold the ingredients in a tablet together. 'Dry binders' are added for example to the powder blend. Preferred dry binders pursuant to the present invention are pharmaceutically acceptable excipients that are selected from the list consisting of methylcellulose, hydroxypropylmethylcellulose, hydroxypropylcellulose, polyvinylpyrrolidone, and vinylpyrrolidone derivatives, such as vinylpyrrolidone-vinyl acetate copolymers, such as KollidonVA64 and KollidonVA64F. KollidonVA64 and KollidonVA64 Fine (KollidonVA64F) are commercially available and are used in pharmaceutical industry as (dry) binder in tablets or granulating agents. A very preferred dry binder is hydroxypropylcellulose.

Preferred types of 'hydroxypropylcellulose' (CAS No. 9004-64-2) as dry binders are hydroxypropylcellulose variants HPC-SSL-SFP and HPC-L due to their favorable properties regarding viscosity, molecular weight and and particle size.

Commercially available super fine powder variant 'HPC-SSL-SFP' is characterized in having a viscosity of 2 to 3 mPas (at 20°C / 2% aq. Solution), a molecular weight (GPC method) of about 40,000 g/mol and a particle size D50 of about 20 µm (e.g. between 10 and 30 µm), D90 of about 50 µm (e.g. between 40 and 60 µm) respectively.

Hydroxypropylcellulose variant 'HPC-L' is characterized in having a viscosity of 6 to 10 mPas (at 20°C / 2% aq. solution), a molecular weight of about 140.000 g/mol (GPC method) and a particle size D50 of about 160 µm (e.g. between 150 and 170 µm), D90 of about 355 µm (e.g. between 335 and 365) respectively.

Wherever throughout this application the 'molecular weight' is specified in the context of a polymer (e.g. hydroxypropylcellulose), said molecular weight refers to a molecular weight as determined by the GPC method (Gel permeation chromatography) as known in the art.

'Disintegrants' expand and dissolve when wet causing the tablet to break apart in the digestive tract, releasing the active ingredients. Disintegrants suitable in the context of the present invention are those selected from the list consisting of alginic acid, cross-linked polyvinylpyrrolidone, maize starch, modified starch, and starch derivatives such as sodium carboxymethyl starch, cellulose derivatives such as carmellose calcium (carboxymethylcellulose calcium) and croscarmellose-sodium (cross-linked polymer of carboxymethylcellulose sodium).

An example for an 'excipient with disintegration promoting properties' is microcrystalline cellulose, such as Avicel^{®} PH- 101 (CAS No. 9004-34-6).

'Lubricants' prevent ingredients from sticking, e.g. to production equipment. Lubricants that can be used in the formulation according to the present invention are those selected from the list consisting of magnesium stearate, sodium stearylfumarate, stearic acid, glycerin monostearate, glycerin monobehenate, calcium behenate, hydogenated vegetable fat or oil, polyethylenglycol and talc. Preferred lubricants according to the present invention are those selected from the list consisting of magnesium stearate, stearic acid and talc. A very preferred lubricant is magnesium stearate (CAS No. 557-04-0).

'Flow agents' are compounds which can be added to improve the flow behavior, e.g. of formulations or powders. A preferred flow agent used in the formulation according to the present invention is highly-disperse silica (for example Aerosil^{®}).

As anyone in the field of pharmaceutical formulation technology may be aware of, the referred to functional descriptions of being a 'dry binder', 'disintegrant', 'flow agent', 'lubricant' and 'excipient with disintegration promoting properties' may be assigned to substances depending on their intended purpose in a given formulation. All of the above types of substances are summarized under the genus of being 'excipients' together with the 'fillers' described below. It may therefore come to happen that one of ordinary skill in the art assigns similar or even identical substances to be member of more than one of the above referred to groups of substances. Within the context of the present invention, the functional descriptions of the substances are however intentionally filled with specific substances to have no overlap to clarify their respective property assigned to them.

However, any formulation according to the present invention may additionally comprise any further combination of pharmaceutically suitable excipients as described herein. Such excipients can also be fillers as commonly known in the art, which are organic fillers or inorganic fillers. Among the organic fillers, those fillers can be sugars or sugar alcohols such as mannitol as well as any carbohydrates such as maltodextrine. Among the inorganic fillers, those fillers can be for example calcium hydrogenphosphate or calcium carbonate or any mixtures of the aforementioned organic and/or inorganic fillers thereof.

Fixed-dose tablet formulations according to the current invention may or may not be arranged in the form of a tablet and may or may not be 'coated' as known in the art.

With regards to the coated tablet pursuant to the present invention, it is clarified that such coating is not a part of the formulation of the tablet according to the present invention. Therefore the potential constituents of a coating of a coated tablet according to the present invention are not to be considered excipients pursuant to the foregoing definition within the formulation and therefore the below listed potential coating substance may be partially identical to the substances listed as any of the above referred to excipients of the formulation.

Non limiting examples of pharmaceutically acceptable 'coating substance' are sugars, polymers such as hydroxypropylmethycellullose, hydroxypropylcellulose, polyethylene glycol-polyvinylalcohol-copolymer, polyvinylalcohol, or polymethacrylates, and natural polymers e.g. shellac, pigments such as ferric oxide, titanium dioxide or indigo carmine, plastizisers such as macrogols, tributyl citrate, tributyl acetylcitrate, dibutyl sebacate, triacetin or glycerine monostearate as well as talc.

Furthermore coating substances with additional functional characteristics may be used such as film-forming materials for a modified drug release such as ammonium methacrylate copolymers, cellulose acetate, cellulose acetate butyrate, chitosan, ethylcellulose, polyacrylates, polyvinylacetate or gastric resistant polymers such as cellulose acetate phthalate, hypromellose acetate succinate, hypromellose phthalate, methacrylic acid-methacrylate-copolymers, poly(methacrylate-co-methylmethacrylate-co-methacrylic acid) or polyvinylphthalate.

As used in this document, the expression 'pharmaceutically acceptable' refers to those compounds, coatings, active compounds, materials, compositions, carriers, and/or dosage forms which are, within the scope of sound medical judgment, suitable for use in contact with the tissues of human beings and animals without excessive toxicity, irritation, allergic response, or other problems or complications, commensurate with a reasonable benefit/risk ratio.

'Dry granulation processes' are known in the art. For example a dry granulation process can be used to form granules without a liquid solution because the product granulated may be sensitive to moisture and heat. Forming granules without moisture in a dry granulation process may occur by compacting and densifying the powders. In this process the primary powder particles are aggregated under high pressure. For example and as known in the art, a swaying granulator or a roll compactor can be used for the dry granulation. Examples for conducting dry granulation include inter alia producing a large tablet in a heavy duty tableting press or squeezing the powder between two counter-rotating rollers to produce a continuous sheet or ribbon of material.

'Roller compaction processes' may comprise the following steps: convey powdered material to the compaction area, e.g. with a screw feeder, compact powder between two counter-rotating rolls with applied forces, mill resulting compact to desired particle size distribution. Roller compacted particle are typically dense, with sharp-edged profiles. Preferably, during roller compaction the powdered material is transported by gravity forces or screws into a gap between two counter rotating rolls. Within the gap the material is densified to a compact by the force transmitted from the rolls. Depending on the surface of the used rolls different types of compacts may be generated (e.g. ribbons, briquettes). Using knurled or smooth surfaces of the rolls a compact band is produced, which is called ribbon. In a second step, the grinding step, the produced compacts may be grinded through a sieve to produce granules.

'Dry mixing' generally refers to mixing at least two materials or compounds, for example by adding the two or more materials or compounds into a drum or container, which will be rotated afterwards for blending.

### Description of the Figures

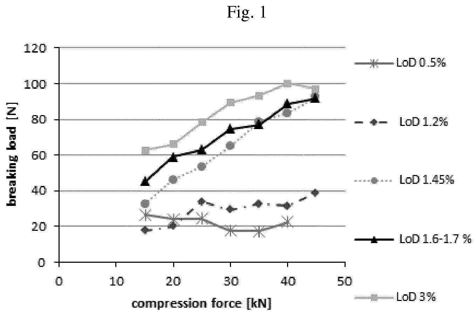

**Figure 1** shows the relationship between moisture content, compression force and breaking load for formulation 4^{∗}. Formulation 4^{∗} is the formulation 4 according to example 3 comprising 4 mg magnesium stearate/tablet instead of 3.5 mg magnesium stearate/tablet. A compression simulator was used for tableting and the profile of the rotary die press FE 55, with a speed of 100.000 tab/h was used. Optimal behavior is observed for a LoD of >1.6%.

### Embodiments

Any tablet formulation according to the present invention, as set out in the appended claims, was surprisingly found to have a dramatically decreased tendency of capping when the formulation is arranged or pressed into the form of those tablets (see table 4).

According to a first aspect of the present invention this is achieved with a fixed dose tablet formulation comprising metformin and acarbose, at least one type of a hydroxpropylcellulose as at least one dry binder, at least one disintegrant, at least one flow agent and/or lubricant, **characterized in that** the at least one type of a hydroxypropylcellulose has a viscosity of 2 to3 mPas at a molecular weight of about 40,000 g/mol and a particle size D₅₀ of about 20 µm, and/or D₉₀ of 50 µm respectively. In a preferred embodiment according to the first aspect the at least one type of a hydroxypropylcellulose is HPC-SSL-SFP. In the same or different preferred embodiments according to the first aspect the moisture content of said formulation is 1.6 to 3.0%, e.g. 1.7 to 2.0%, e.g. 1.8 to 1.9%, e.g. 1.7 or 1.8%.

According to a second aspect of the present invention a decreased tendency of capping is achieved by providing a fixed dose tablet formulation comprising metformin and acarbose, at least one type of a vinylpyrrolidone derivative as at least one dry binder, at least one disintegrant, at least one flow agent and/or lubricant, characterized in that the moisture content of said formulation is above 1.9%. In a preferred embodiment according to the second aspect the vinylpyrrolidone derivative is a vinylpyrrolidone-vinyl acetate copolymer such as Kollidon, such as Kollidon VA64 or VA64F. According to a third aspect of the present invention there is provided a fixed dose tablet formulation comprising metformin and acarbose, at least one type of a hydroxpropylcellulose as at least one dry binder, at least one disintegrant, at least one flow agent and/or lubricant, characterized in that the moisture content of said formulation is 1.6 to 3.0%, e.g. 1.7 to 2.0%. In some preferred embodiments according to the third aspect the moisture content of said formulation is 1.8 to 1.9% and in a most preferred embodiment according to the third aspect the moisture content of said formulation is about 1.7 or 1.8%.

Where the moisture content of the formulation is tightly controlled as described for the current invention it was surprisingly found that the metformin/acarbose formulation showed beneficial behavior with regard to stability, capping and release of active ingredients, while degradation of active ingredients such as metformin and acarbose could be avoided (see tables 4, 5, 6 and figure 1).

Therefore according to one aspect of the present invention there is provided a fixed dose tablet formulation comprising metformin and acarbose, at least one dry binder, at least one disintegrant, at least one flow agent and/or lubricant, characterized in that the moisture content of said formulation is 1.6% to 3.0%.

In a preferred embodiment according to the first, second, third or any other aspect or mentioned embodiment of the current invention the formulation comprising metformin and acarbose is characterized in having an acarbose to metformin weight ratio of about 1 to 10 (e.g. 50 mg acarbose with 500 mg metformin), or about 1 to 8.5 (e.g. 100 mg acarbose with 850 mg metformin) or about 1 to 5 (e.g. 100 mg acarbose with 500 mg metformin). In other embodiments according to the first, second, third or any other aspect or mentioned embodiment of the current invention the formulation comprising metformin and acarbose is characterized in having an acarbose to metformin weight ratio of about 1 to 17 or 1 to 12.5.

In a preferred embodiment which is the same or different from the aforementioned embodiments, according to the first, second, third or any other aspect of the current invention the fixed dose tablet formulation is characterized in being arranged in the form of a tablet, e.g. in the form of a tablet that has 18 mm length, a width of about 8 mm and a thickness of about 6.3 - 7 mm. In a preferred embodiment which is the same or different from the aforementioned embodiments, according to the first, second, third or any other aspect of the current invention the fixed dose tablet formulation is characterized in being arranged in the form of a tablet, e.g. in the form of a tablet that has 18 mm length, a width of about 8 mm and a thickness of about 6.3 - 7 mm, wherein the resulting form of a tablet is furthermore coated.

Particularly preferred according to the current invention and in particular according to the first or second aspect and the aforementioned embodiments are formulations comprising the above referred to at least one hydroxypropylcellulose and another hydroxypropylcellulose as a dry binder that has a viscosity of 6 to 10 mPas at a molecular weight of about 140,000 g/mol and a particle size D₅₀ of about 160 µm, and/or D₉₀ of 335 µm or 355 µm respectively.

Particularly preferred according to the current invention and in particular according to the first or second aspect and the aforementioned embodiments are formulations comprising the above referred to at least one hydroxypropylcellulose and another hydroxypropylcellulose as a dry binder that has a viscosity of 6 to 10 mPas at a molecular weight of about 140,000 g/mol and a particle size D₅₀ of about 160 µm, and/or D₉₀ of 335 µm or 355 µm respectively, wherein the moisture content of said formulation is 1.6 to 3.0%, e.g. 1.7 to 2.0%, e.g. 1.8 to 1.9%, e.g. 1.7 or 1.8%.

In some of these aforementioned particularly preferred embodiments the another hydroxypropylcellulose is HPC-L.

It has surprisingly been found that the combination of both types of hydroxypropylcellulose has the above referred to advantageous properties of the tablet being pressed thereof showing no capping and additionally has an improved disintegration and release pattern (see tables 2, 3 and 4).

It has furthermore surprisingly been found that the beneficial impact of combining the two types of hydroxypropylcellulose could be increased by adjusting the moisture content as described above (see table 5 and 6).

Such combined advantage of faster disintegration/release and no capping synergizes to allowing to even further reduce the overall amount of excipients while resulting in a formulation that is good to be tableted and fast disintegrating/releasing, if the ratio of the hydroxypropylcellulose with a viscosity of 2 to 3 mPas at a molecular weight of about 40,000 g/mol and a particle size D₅₀ of about 20 µm, and/or D₉₀ of 50 µm respectively towards the hydroxypropylcellulose with a viscosity of 6 to 10 mPas at a molecular weight of about 140,000 g/mol and a particle size D₅₀ of about 160 µm, and/or D₉₀ of 335 µm or 355 µm respectively is or is adjusted to a range between 4:1 and 1:1, particularly to about 3:1.

Particularly preferred according to the current invention and in particular according to the first or third aspect and the aforementioned embodiments are formulations comprising
a) the above referred to at least one hydroxypropylcellulose characterized in having a viscosity of 2 to 3 mPas at a molecular weight of about 40,000 g/mol and a particle size D₅₀ of about 20 µm, and/or D₉₀ of 50 µm respectively, and
b) another hydroxypropylcellulose as a dry binder characterized in having a viscosity of 6 to 10 mPas at a molecular weight of about 140,000 g/mol and a particle size D₅₀ of about 160 µm, and/or D₉₀ of 335 µm or 355 µm respectively,
c) wherein the ratio between said at least one hydroxypropylcellulose and said another hydroxypropylcellulose is between 4:1 and 1:1 (e.g. 3:1), and
d) wherein the moisture content of said formulation is 1.6 to 3.0%, e.g. 1.7 to 2.0%, e.g. 1.8 to 1.9%, e.g. 1.7 or 1.8%.

According to some preferred embodiments of the present invention, the above referred to at least one hydroxypropylcellulose with a viscosity of 2 to 3 mPas at a molecular weight of about 40,000 g/mol and a particle size D₅₀ of about 20 µm, and/or D₉₀ of 50 µm respectively as a dry binder is present in the formulation in an amount from 2.5% to 10% by weight, preferably in an amount from 7% to 9%, more preferably in an amount of about 8% by weight. These embodiments are compatible and suggested to be combined with each embodiment comprising said at least one hydroxypropylcellulose.

According to further preferred embodiments of the present invention, the above referred to hydroxypropylcellulose with a viscosity of 6 to10 mPas at a molecular weight of about 140,000 g/mol and a particle size D₅₀ of about 160 µm, and/or D₉₀ of 335 µm or 355 µm respectively as a dry binder is present in the formulation in an amount from about 1.5% to 5% by weight - preferably in an amount from about 1.5% to 3.5% by weight, preferably in an amount from about 2% to 3%, more preferably in an amount of about 2.5 or 2.7% by weight - or in an amount from about 2% to 4% by weight. These embodiments are compatible and suggested to be combined with each embodiment comprising said hydroxypropylcellulose with a viscosity of 6 to10 mPas.

The dry binders are preferably present in the formulation in an overall amount from about 5% to 15% by weight, more preferably from about 7% to 12% by weight, even more preferably from about 8% to 11% by weight.

According to some preferred embodiments according to the first, the second, third or any other aspect according to the current invention the formulation further comprises microcrystalline cellulose, such as Avicel, e.g. as an excipient with disintegration promoting properties.

According to some different or same preferred embodiments of the present invention, microcrystalline cellulose as an excipient with disintegration promoting properties is present in the formulation in an amount from 5% to 15% by weight, preferably in an amount from 8% to 12%, more preferably in an amount of about 9% by weight.

Excipients with disintegration promoting properties such as microcrystalline cellulose may be omitted in the very preferred formulations of the present invention that have the above referred to hydroxypropylcellulose with a viscosity of 2 to 3 mPas at a molecular weight of about 40,000 g/mol and a particle size D₅₀ of about 20 µm, and/or D₉₀ of 50 µm respectively as well as the hydroxypropylcellulose with a viscosity of 6 to10 mPas at a molecular weight of about 140,000 g/mol and a particle size D₅₀ of about 160 µm, and/or D₉₀ of 335 µm or 355 µm, respectively at a range between 4:1 and 1:1, as those procure that the formulation maintains all relevant positive properties even in absence of such usually added excipients with disintegration promoting properties.

Addition of excipients with disintegration promoting properties such as microcrystalline cellulose, however further helps to improve the long time stability of the disintegration properties of tablets formed out of such formulations.

Formulations according to the present invention contain at least one disintegrant. Preferred disintegrants pursuant to the present invention are those selected from the list consisting of sodium starch glycolate (such as, for example, Explotab^{®}), cross-linked polyvinylpyrrolidone (such as Polyplasdone^{®}) and croscarmellose-sodium (such as, for example, AcDiSol^{®}). Very preferred disintegrants are cross-linked polyvinylpyrrolidones (such as Polyplasdone^{®}) and/or croscarmellose-sodium (such as AcDiSol^{®}). Particularly preferred disintegrants are cross-linked polyvinylpyrrolidones (such as Polyplasdone^{®}).

Those particularly preferred disintegrants being cross-linked polyvinylpyrrolidones are available at different grades, differentiated by particle size. If sold under the above referred to brand name "Polyplasdone^{®}", three grades from coarse to fine can be discriminated. Those grades are Polyplasdone^{®} XL, Polyplasdone^{®} XL-10 and Polyplasdone^{®} INF-10 and correspond - in the same order - to average particle sizes of about 100µm, about 30µm and about 11µm.

A particularly preferred formulation pursuant to the present invention comprises cross-linked polyvinylpyrrolidone with an average particle size of about 30µm as a disintegrant.

For particularly preferred embodiments pursuant to the present invention the at least one disintegrant is cross-linked polyvinylpyrrolidone with an average particle size of about 30µm, e.g. in an amount from 2.5% to 7.5% by weight, preferably in an amount from 3.5% to 5.5%, more preferably in an amount of about 4.5% by weight. According to the very preferred embodiments of the present invention, cross-linked polyvinylpyrrolidone or croscarmellose-sodium as a disintegrant are present in the formulation in an amount from 2.5% to 7.5% by weight, preferably in an amount from 3.5% to 5.5%, more preferably in an amount of about 4.5% by weight.

According to the particularly preferred embodiments of the present invention, cross-linked polyvinylpyrrolidone with an average particle size of about 30µm is used as a disintegrant in an amount from 2.5% to 7.5% by weight, preferably in an amount from 3.5% to 5.5%, more preferably in an amount of about 4.5% by weight.

Furthermore formulations according to the present invention contain at least one lubricant and/or flow agent.

Preferred lubricants according to the present invention are those selected from the list consisting of magnesium stearate, stearic acid and talc. A very preferred lubricant is magnesium stearate.

A preferred flow agent used in the formulation according to the present invention is highly-disperse silica (for example Aerosil^{®}).

If used in combination, a preferred combination of lubricant and flow agent is magnesium stearate and highly-disperse silica, such as Aerosil.

In a preferred embodiment of this invention the formulation comprises at least one flow agent and at least one lubricant.

In an equally preferred embodiment of this invention the formulation only comprises a lubricant, which preferably is magnesium stearate.

Preferred amounts of lubricants present in the formulation according to the present invention are amounts of from 0.1% to 3% by weight, more preferably amounts from 0.25% to 0.75%, even more preferably amounts of about 0.5% by weight.

If present, preferred amounts of flow agents are amounts of from 0.1% to 1% by weight, more preferably amounts from 0.25% to 0.75%, even more preferably amounts of about 0.5% by weight.

If flow agents and lubricants are both present in the formulation according to the present invention, the overall amount of flow agents and lubricants therefore is preferably from 0.2% to 4% by weight, preferably each in an amount from about 0.25% to 0.75%, more preferably each in an amount of about 0.5% by weight.

According to a likewise preferred embodiment of the present invention the flow agent is highly-disperse silica present in the formulation in an amount of from 0.1% to 1% by weight, preferably in an amount from 0.25% to 0.75%, more preferably in an amount of about 0.5% by weight, and the lubricant is magnesium stearate present in the formulation in an amount of from 0.1% to 3% by weight, preferably in an amount from 0.25% to 0.75%, more preferably in an amount of about 0.5% by weight.

Particularly preferred according to the current invention and in particular according to the first or third aspect and the aforementioned embodiments are formulations
a) comprising the above referred to at least one hydroxypropylcellulose characterized in having a viscosity of 2 to 3 mPas at a molecular weight of about 40,000 g/mol and a particle size D₅₀ of about 20 µm, and/or D₉₀ of 50 µm respectively, and
b) comprising another hydroxypropylcellulose as a dry binder characterized in having a viscosity of 6 to 10 mPas at a molecular weight of about 140,000 g/mol and a particle size D₅₀ of about 160 µm, and/or D₉₀ of 335 µm or 355 µm respectively,
c) wherein the ratio between said at least one hydroxypropylcellulose and said another hydroxypropylcellulose is between 4:1 and 1:1 (e.g. 3:1), and
d) further comprising
   a. cross-linked polyvinylpyrrolidone (e.g. with 2.5% to 7.5% by weight), e.g. with an average particle size of about 30 µm as a disintegrant and/or
   b. highly-disperse silica (e.g. with 0.1% to 1% by weight) and/or magnesium stearate (e.g. with 0.1% to 3% by weight) as the at least one flow agent and/or lubricant,
e) wherein the moisture content of said formulation is 1.6 to 3.0%, e.g. 1.7 to 2.0%, e.g. 1.8 to 1.9%, e.g. 1.7 or 1.8%.

Particularly preferred according to the current invention and in particular according to the second, first or third aspect and the aforementioned embodiments are formulations
a) comprising cross-linked polyvinylpyrrolidone (e.g. with 2.5% to 7.5% by weight), e.g. with an average particle size of about 30 µm as a disintegrant and/or
b) comprising highly-disperse silica (e.g. with 0.1% to 1% by weight) and/or magnesium stearate (e.g. with 0.1% to 3% by weight) as the at least one flow agent and/or lubricant.

The formulation according to the present invention is a fixed dose formulation of acarbose and metformin and thus the two actives are present in the formulation of the present invention in a predetermined ratio.

The ratio of acarbose to metformin can be - from a formulation perspective - any therapeutically effective ratio of the two actives. In a preferred embodiment of the present invention, the ratio of acarbose to metformin is 1:10 or 1:5.

In a very preferred embodiment of the present invention the formulation comprises an overall amount of at least 60% by weight of the actives in a ratio of 1:10 between acarbose and metformin.

In an even more preferred embodiment of the present invention the formulation comprises an overall amount of at least 70% by weight of the actives in a ratio of 1:10 between acarbose and metformin. Most preferably the formulation according to the present invention comprises acarbose and metformin in a ratio of 1:10 while the overall amount of these two actives is about 74% by weight.

Particularly preferred according to the current invention and in particular according to the first or second aspect and the aforementioned embodiments are formulations
a) comprising acarbose and metformin at a weight ratio between 1 to 17 and 1 to 4 (e.g. of about 1 to 12.5, about 1 to 10, or about 1 to 8.5 or about 1 to 5),
b) wherein optionally the overall amount of the actives is at least 60% (e.g. at least 70% or about 74%) by weight of said formulation,
c) comprising the above referred to at least one hydroxypropylcellulose characterized in having a viscosity of 2 to 3 mPas at a molecular weight of about 40,000 g/mol and a particle size D₅₀ of about 20 µm, and/or D₉₀ of 50 µm respectively, and
d) comprising another hydroxypropylcellulose as a dry binder characterized in having a viscosity of 6 to 10 mPas at a molecular weight of about 140,000 g/mol and a particle size D₅₀ of about 160 µm, and/or D₉₀ of 335 µm or 355 µm respectively,
e) wherein the ratio between said at least one hydroxypropylcellulose and said another hydroxypropylcellulose is between 4:1 and 1:1 (e.g. 3:1), and
f) optionally further comprising
   a. cross-linked polyvinylpyrrolidone (e.g. with 2.5% to 7.5% by weight), e.g. with an average particle size of about 30 µm as a disintegrant and/or
   b. highly-disperse silica (e.g. with 0.1% to 1% by weight) and/or magnesium stearate (e.g. with 0.1% to 3% by weight) as the at least one flow agent and/or lubricant,
g) wherein the moisture content of said formulation is 1.6 to 3.0%, e.g. 1.7 to 2.0%, e.g. 1.8 to 1.9%, e.g. 1.7 or 1.8%.

Particularly preferred according to the current invention and in particular according to the second, first, third or any other aspect and the aforementioned embodiments are formulations
a) comprising acarbose and metformin at a weight ratio between 1 to 17 and 1 to 4 (e.g. of about 1 to 12.5, about 1 to 10, or about 1 to 8.5 or about 1 to 5),
b) wherein optionally the overall amount of the actives is at least 60% (e.g. at least 70% or about 74%) by weight of said formulation,
c) further comprising cross-linked polyvinylpyrrolidone (e.g. with 2.5% to 7.5% by weight), e.g. with an average particle size of about 30 µm as a disintegrant and/or
d) highly-disperse silica (e.g. with 0.1% to 1% by weight) and/or magnesium stearate (e.g. with 0.1% to 3% by weight) as the at least one flow agent and/or lubricant.

As it can be seen from the most preferred embodiment of the present invention, the relative amount of excipients compared to the two actives is comparably low. This facilitates that the overall tablet size can be kept low as well.

In an outmost preferred embodiment of the present invention, the formulation comprises at least
a) about 74% (e.g. between 67 and 77%) by weight of acarbose and metformin in a ratio of 1:10 or 1:5,about 10.5% (e.g. between 9.8 and 11.5%) by weight of dry binders,
   a. consisting of about 2.5% (e.g. 2.7%) by weight of hydroxypropylcellulose with a viscosity of 6 to10 mPas, a molecular weight of about 140,000 g/mol and a particle size D₅₀ of about 160 µm, and/or D₉₀ of 335 µm or 355 µm respectively and
   b. about 8% (e.g. between 8 and 12%) by weight of hydroxypropylcellulose with a viscosity of 2 to3 mPas, a molecular weight of about 40,000 g/mol and a particle size D₅₀ of about 20 µm, and/or D₉₀ of 50 µm respectively
b) about 9% (e.g. between 7 and 13%, e.g. 9.4%) by weight of microcrystalline cellulose as an excipient with disintegration promoting properties,
c) about 4.5% by weight of a disintegrant (e.g. between 4.1 and 4.95%), preferably being cross-linked polyvinylpyrrolidone with an average particle size of about 30µm, and
d) about 0.5% by weight of both a lubricant and a flow agent, wherein the flow agent is preferably highly-disperse silicas and the lubricant is preferably magnesium stearate.

As may be seen just above, the overall amount of ingredients of the outmost preferred formulation pursuant to the present invention do not sum up to 100% by weight, but only 98.5% by weight. This is because the above referred to amounts are specified to be 'about' the values that are mentioned and because these values comprise rounding errors. Everyone of ordinary skill in the art should however be able to produce a formulation according to the present invention that intrinsically sums up to 100% by weight and satisfies the herein mentioned limitations while achieving the beneficial effects associated therewith without undue burden, when just referring to the embodiments disclosed just above.

For sake of clarification, a proportion or value characterized to be 'about' a value, is always to be understood to also comprise any value lesser or higher than the actually stated value that allows the overall formulation to result in 100% by weight. Mostly the deviation around the explicitly stated values should not be more than 25% of the specified value to allow that.

As a matter of example, the above referred to rounding errors resulting in the ingredients of the outmost preferred embodiment of the present invention to sum up to only about 98.5% may be compensated by adding any of the above fillers or further excipients not specifically mentioned.

The tablets produced from a formulation pursuant to the preferred embodiments of the present invention combine all beneficial effects that have been referenced before.

According to a fourth aspect of the current invention there is provided a tablet comprising a formulation according to any of the aforementioned aspects. Those tablets formed from the formulation according to the present invention, form another aspect of the present invention.

Tablets pursuant to the present invention display no capping when produced even under high compaction forces and high tableting speed, show fast disintegration and thus rapid release of both actives, and are comparably small with regards to the overall amount of actives contained therein. With regard to disintegration, this is to be understood pursuant to the present invention to be a disintegration time measured pursuant to European Pharmacopoeia (2017) section 9.2. Obviously a shorter time is indicative for a faster disintegration and a longer time is indicative for inferior disintegration.

As particularly metformin is a substance that is to be used in the treatment of Type 2 Diabetes induced hyperglycemia, which may under sever conditions be live threatening per se, a comparably fast release of the Metformin to reduce the blood sugar level of the patient suffering from acute hyperglycemia back to a tolerable level is advantageous.

For a different reason, the same applies for the release of acarbose from the tablet. Acarbose is usually applied postprandial to mitigate the risk of hyperglycemia subsequent to uptake of food of a given Type 2 Diabetes patient by partial inhibition of a-glycosidases. To efficiently allow said effect, an immediate release of Acarbose administered via postprandial tablet intake is required.

The release of acarbose and/or metformin into the patient from a tablet made up from a formulation pursuant to the present invention is linked to above referred to disintegration time, but said link is not necessarily linear. In principle, however, a fast release is usually linked to an also fast disintegration, while fast disintegration does not necessarily result in an as fast as anticipated release.

Tablets according to the fourth aspect, e.g. made up from the formulation pursuant to the first, second or third aspect and the respective embodiments as described just above within this invention, share the positive effect of collectively having a disintegration time of less than 15 min. and display no capping.

Those tablets made of formulations pursuant to preferred embodiments of the invention have disintegration times of below 15 min and display no capping.

Resulting therefrom those tablets release more than 60% in preferred embodiments more than 80% of the labeled amount of both actives after 30 min. Tablets pursuant to the present invention may be in whatever kind of form that is deemed to be advantageous.

As outlined above, a fixed dose acarbose and metformin tablet that has 18 mm length, a width of about 8 mm and a thickness of about 6.3 - 7 mm is known to be well accepted on the Asian market as of being comparably easy to swallow while it comprises the necessary amount of active to result in sufficient Diabetes Type 2 efficacy.

The tablets according to the present invention may be coated or not. It is however preferred that the tablet according to the present invention is coated. According to the present invention "tablet" means the non-coated core.

In some preferred embodiments according to the fourth aspect, this invention therefore comprises a coated tablet comprising the above referred to tablet as a core that is made from any of the before described fixed dose tablet formulations according to the first, second or third aspect of the invention, **characterized in that** such tablet is coated.

The coating of the coated tablet pursuant to the third aspect of this invention may be any coating material that is pharmaceutically acceptable.

A mixture of the coating substances mentioned herein may also be used as a ready-to-use coating system such as commercially available under the trade name Opadry^{®}.

Opadry 14F220001^{®} is a mixture of about 60 wt.% hydroxypropylmethylcellulose, about 16 wt.% titanium dioxide, about 4 wt.% ferric oxide yellow and about 20 wt.% polyethylene glycol.

Any coating according to the fourth aspect of the present invention can make up from 0.5% to 30% by weight of the coated tablet including the tablet according to the second aspect of the present invention made up from the formulation of the first aspect of the invention depending on the type of coating chosen. The higher percentage of weight ranges, usually apply to tablets coated with a sugar containing coating. One of ordinary skill in the art, would however than term the coated to be rather a dragee than a coated tablet. For the purpose of the present invention, the coated tablet can therefore be either a dragee or a coated tablet in a more narrow sense.

Preferably the coating is about 0,5% to 3% by weight of the coated tablet formulation, more preferably about 2% by weight of the coated tablet.

A very preferred coating is a coating made up from a mixture of about 60 wt.% hydroxypropylmethylcellulose, about 16 wt.% titanium dioxide, about 4 wt.% ferric oxide yellow and about 20 wt.% polyethylene glycol. If such mixture is used, the coating makes up from 0.5% to 3% by weight of the coated tablet including the tablet of the fourth aspect of the invention made up from the formulation according to the first, second or third aspect of the present invention. Preferably such coating is then about 1% to 2.5% by weight of the coated tablet, more preferably about 2% by weight of the coated tablet.

In a fifth aspect of the present invention, a process for the production of a tablet formulation or tablet according to the first, second, third or fourth aspect of the invention is provided, wherein said process comprises the steps of
1) dry mixing metformin and acarbose together with at least one dry binder and at least one disintegrant,
2) dry granulating the resulting mixture to obtain dry granules,
3) at least once blending said dry granules with at least one flow agent and/or lubricant to obtain the fixed dose combination formulation of acarbose and metformin,
4) tableting said fixed dose combination formulation of acarbose and metformin to become a tablet, e.g. with tableting forces of at least 7 kN using circumferential speeds of the punches of at least 0.15 m/s or producing at least 20,000 tablets/h, whichever is lower.

Said process according to the fifth aspect of the present invention, preferably comprises another step of 5) coating the tablet of step 4) e.g. to result in the coated tablet according to the fourth aspect of the present invention.

Within the process of the fifth aspect of the present invention, it is preferred that the dry granulation performed pursuant to step 2) of the process is done as a roller compaction, e.g. with a throughput of at least 20 kg/h. In a preferred embodiment, the process according to the fifth aspect is a dry granulation process. In a preferred embodiment, dry granulation of the material comprises use of a roller compaction process. According to some embodiments according to the current invention roller compaction is preferably done with a throughput of at least 20 kg/h.

It has surprisingly been found that the above referred to process allows to produce tablets of the fixed dose combination of acarbose and metformin formulation without having to add water. That is to say that true dry granulation instead of either elevated temperature (melt) granulation or wet granulation can be used, which is particularly advantageous, as such process addresses the (negative) hygroscopic properties of both actives as well as the poor thermal stability of acarbose.

Furthermore the above process is capable to produce tablets in high amounts in continuous manufacturing, which comes along with meeting the above referred to objective to provide tablets to a huge market at affordable prizes.

However, with regard to the formulations and the processes described herein it was found that a tight control of the water amount at different stages of the production process and in particular of the final formulation or tablet was beneficial to avoid capping of the tablets. These investigations showed that a certain amount of water within the formulation (Loss on Drying ≥ 1.6%) was beneficial to avoid capping of tablets comprising the fixed dose combination formulation and thus to obtain a more stable and reliable high-throughput production process.

According to a sixth aspect, there is provided a process for the production of a fixed dose tablet formulation according to the first, second or third or another aspect comprising the steps of
1) dry mixing metformin and acarbose, optionally together with the at least one dry binder and/or at least one disintegrant,
2) dry granulating the resulting mixture to obtain dry granules,
3) optionally at least once blending said dry granules with a further excipient, e.g. the at least one flow agent and/or lubricant to obtain the fixed dose tablet formulation of acarbose and metformin,
4) determining the moisture content of the formulation and/or the dry granules and
5) adding water if the formulation moisture content as determined is below 1.6% loss on drying (LoD), e.g. to reach a formulation target moisture of 1.6 to 3.0%, e.g. 1.7 to 2.0%, 1.8 to 1.9%, 1 .7% or 1.8%, and
6) optionally arranging the fixed dose combination formulation in the form of a tablet (tableting), and
7) optionally coating the tablet obtained from step 5.

In a preferred embodiment, the order of steps according to the sixth aspect is step 1, step 4, step 2, step 5, step 3, step 6 and step 7. However the person skilled in the art easily recognizes that the order of steps can be changed and other sequences of the steps are possible according to the present invention.

According to a seventh aspect, there is provided a process wherein said process comprises the steps of
1) dry mixing metformin and acarbose together with at least one dry binder and at least one disintegrant,
2) dry granulating the resulting mixture to obtain dry granules,
3) at least once blending said dry granules with at least one flow agent and/or lubricant to obtain a fixed dose combination formulation of acarbose and metformin,
4) determining the moisture content of the formulation and/or the dry granules and
5) adding water if the formulation moisture content as determined is below 1.6% LoD, e.g. to reach a formulation target moisture of 1.6 to 3.0%, and optionally
6) tableting said fixed dose combination formulation of acarbose and metformin to become a tablet,
**characterized in that** said at least one dry binder is a hydroxypropylcellulose that has a viscosity of 2 to 3 mPas at a molecular weight of about 40,000 g/mol and a particle size D₅₀ of about 20 µm, and/or D₉₀ of 50 µm respectively.

In a preferred embodiment, the order of steps according to the seventh aspect is step 1, step 4, step 2, step 5, step 3 and step 6. However the person skilled in the art easily recognizes that the order of steps can be changed and other sequences of the steps are possible according to the present invention.

In a preferred embodiment according to the fifth, sixth and seventh aspect tableting occurs with tableting forces of at least 7 kN using circumferential speeds of the punches of at least 0.15 m/s or producing at least 20,000 tablets/h whichever is lower.

Monitoring/determining the moisture content of the formulation, e.g. the tight control of water content can be performed by analyzing the amount of water within the material as known in the art. For example, loss on drying (LOD) is a method to measure high level moisture (moisture content (MC)) in solid or semi-solid materials. As known in the art for this technique a sample of material or of the formulation is weighed, heated in an oven for an appropriate period, cooled in the dry atmosphere e.g. of a desiccator, and then reweighed. If the volatile content of the solid is primarily water, the LOD technique gives a good measure of moisture content. Where the moisture content or LoD is given as a percentage throughout this application the stated value refers to the specific value with an error of +/-0.05%. Because the manual laboratory method is relatively slow, automated moisture analyzers have been developed that can reduce the time necessary for a test from a couple hours to just a few minutes. These analyzers incorporate an electronic balance with a sample tray and surrounding heating element. Under microprocessor control the sample can be heated rapidly and a result computed prior to the completion of the process, based on the moisture loss rate, known as a drying curve.

The amount of water depends on various factors and even where the process was highly standardized, the amount of water was found to vary between different production sites (e.g. different geographic regions or countries) and different production scales (e.g. lab scale and large scale for commercial production). While standardization of the environmental conditions and process conditions as described herein could improve the capping behavior of the tablets in general, in particular for production sites located in Asia the capping behavior could be furthermore improved by control of the water content of the formulation, mixture, granules, or tablets. While the critical lower limit for LoD was found to be 1.5% no sticking was observed up to a LoD of 3%. Capping could be largely avoided by adding water where the moisture content was found to be lower or equal to 1.6%.

Where the amount of water was found to be too low in the material, e.g. in the formulation, granules or the tablet, water can be added, e.g. to the granules, formulation, material or the tablet, to reach a specific amount of moisture content. The skilled person is well aware that the order of process steps may be varied and that for example adding water may occur at different time points. In some preferred embodiments e.g. according to the sixth or seventh aspect, adding water occurs by spraying. Preferably, for addition of the water, the water is sprayed onto the powdered material or the granules. For example, the spraying can be performed manually by using a commercially available pump spray, thereby adding water into the chamber. For example, the water is thereby sprayed on top of the material and the material is blended in between and afterwards manually or by using a blending machine.

Moreover, the spraying can for example be performed automatically, e.g. by using a spraying system consisting of a spray nozzle for nebulizing the water, a funnel, a pump and a reservoir for the water. The water is transported by the pump, e.g. out of the reservoir through the funnel into the spray nozzle and is subsequently sprayed out of the nozzle onto the material or granules. For example, water spraying can be performed either directly into a container comprising the material (e.g. powdered or as a batch of granules) or directly into a flow of granules.

Where spraying occurs directly into the container or drum, the water is preferably brought into a uniform distribution. To this end the container/drum may be rotated for blending. Where spraying occurs into a granule flow, the water is preferably brought into a uniform distribution, which can for example be reached by moving the material from a delivering unit (e.g. a drum, container, sieving machine, roller compactor) into a receiving unit (e.g. drum, container). For example this step may be carried out while the spraying process takes place or afterwards.

In a preferred embodiment according to the fifth, sixth or seventh aspect the process comprises determining the moisture content of the material and, optionally, where the moisture content is below 1.6%, adding water to the material e.g. powder, granules, mixture, formulation or tablet. Determining the moisture content can be performed as known in the art, preferably by LoD.

In a preferred embodiment according to the fifth, sixth or seventh aspect adding water occurs by spraying water. In a preferred embodiment according to the fifth or sixth aspect adding water occurs by adding water for a target moisture content for the material e.g. granules, formulation or tablet of 1.6 to 3.0%, e.g. 1.7 to 2.0%, 1.8 to 1.9% or 1.7 or 1.8%.

In a preferred embodiment according to the fifth, sixth or seventh aspect adding water occurs by spraying water, e.g. as described above, to reach a target moisture content for the material or tablet of 1.6 to 3.0%,, e.g. 1.7 to 2.0%, 1.8 to 1.9% or 1.7% or 1.8%.

According to an eighth aspect, there is provided a fixed dose tablet formulation produced by a process according to the fifth, sixth or seventh aspect of the current invention.

The present invention further comprises the aspects defined in the following clauses.

According to clause 1 there is provided a fixed dose tablet formulation comprising metformin and acarbose, at least one type of a hydroxpropylcellulose as at least one dry binder, at least one disintegrant, at least one flow agent and/or lubricant, characterized in that the at least one type of a hydroxypropylcellulose has a viscosity of 2 to 3 mPas at a molecular weight of about 40,000 g/mol and a particle size D₅₀ of about 20 µm, D₉₀ of 50 µm respectively

According to clause 2 there is provided a fixed dose tablet formulation according to clause 1, wherein the formulation comprises another hydroxypropylcellulose as a dry binder that has a viscosity of 6-10 mPas at a molecular weight of about 140,000 g/mol and a particle size D₅₀ of about 160 µm, D₉₀ of 335 µm respectively.

According to clause 3 there is provided a fixed dose tablet formulation according to clause 2, wherein the ratio of the hydroxypropylcellulose with a viscosity of 2-3 mPas at a molecular weight of about 40,000 g/mol and a particle size D₅₀ of about 20 µm, D₉₀ of 50 µm respectively towards the hydroxypropylcellulose with a viscosity of 6-10 mPas at a molecular weight of about 140,000 g/mol and a particle size D₅₀ of about 160 µm, D₉₀ of 335 µm respectively is between 4:1 and 1:1, particularly to about 3:1.

According to clause 4 there is provided a fixed dose tablet formulation according to any one of the previous clauses, wherein the dry binders are present in the formulation in an overall amount from about 4% to 15.5% by weight, more preferably from about 9% to 13% by weight, even more preferably from about 8% to 11%.

According to clause 5 there is provided a fixed dose tablet formulation according to any one of the previous clauses, wherein cross-linked polyvinylpyrrolidone is the disintegrant.

According to clause 6 there is provided a fixed dose tablet formulation according to any one of the previous clauses, wherein said formulation magnesium stearate as a lubricant.

According to clause 7 there is provided a fixed dose tablet formulation according to any one of the previous clauses, further comprising microcrystalline cellulose as an excipient with disintegration promoting properties.

According to clause 8 there is provided a fixed dose tablet formulation according to any one of the previous clauses, wherein the formulation comprises about 74% by weight of acarbose and metformin in a ratio of 1:10, about 10.5% by weight of dry binders, consisting of about 2.5% by weight of hydroxypropylcellulose with a viscosity of 6-10mPas, a molecular weight of about 140,000 g/mol and a particle size D50 of about 160 µm, D90 of 335 µm respectively and about 8% by weight of hydroxypropylcellulose with a viscosity of 2-3 mPas, a molecular weight of about 40,000 g/mol and a particle size D50 of about 20 µm, D90 of 50 µm respectively, about 9% by weight of microcrystalline cellulose as an excipient with disintegration promoting properties about 4.5% by weight of a disintegrant, preferably being cross-linked polyvinylpyrrolidone with an average particle size of about 30µm, and about 0.5% by weight of both a lubricant and a flow agent, wherein the flow agent is preferably highly-disperse silicas and the lubricant is preferably magnesium stearate.

According to clause 9 there is provided a tablet comprising a fixed dose tablet formulation of any one of the previous clauses.

According to clause 10 there is provided a tablet according to clause 9, wherein said tablet has a length of about 18 mm, a width of about 8 mm and a thickness of about 6.3 - 7 mm.

According to clause 11 there is provided a coated tablet comprising a core made up from a tablet according to any one of clauses 9 to 10 characterized in that such core is coated.

According to clause 12 there is provided a coated tablet according to clause 11, wherein the coating is a mixture of about 60 wt.% hydroxypropylmethylcellulose, about 16 wt.% titanium dioxide, about 4 wt.% ferric oxide yellow and about 20 wt.% polyethylene glycol.

According to clause 13 there is provided a process for the production of a tablet according to any one of the clauses 9 or 10, wherein said process comprises the steps of
1) dry mixing metformin and acarbose together with at least one dry binder and at least one disintegrant,
2) dry granulating the resulting mixture to obtain dry granules,
3) at least once blending said dry granules with at least one flow agent and/or lubricant to obtain the fixed dose combination formulation of acarbose and metformin,
4) tableting said fixed dose combination formulation of acarbose and metformin to become a tablet,
**characterized in that** said at least one dry binder is a hydroxypropylcellulose that has a viscosity of 2-3 mPas at a molecular weight of about 40,000 g/mol and a particle size D₅₀ of about 20 µm, D₉₀ of 50 µm respectively.

According to clause 14 there is provided a process for the production of a coated tablet, wherein said process comprises the process according to Claim 13 and further comprises another step of
5) coating the tablet after step 4) to result in the coated tablet according to any one of clauses 11 and 12.

### Examples:

### Example 1: Process for production of fixed dose composition tablet formulation and methods for analysis

Excipients and active pharmaceutical ingredients (APIs) were blended. Afterwards these powder blends were compacted with an instrumented roller compactor and grinded. Subsequently the granules were blended with the flow agent and the lubricant. This ready-to-press blend was compressed on a rotary die press as well as on a compression simulator to obtain oval tablets of different sizes (18×8, 19×9 mm) and masses between 712.5 and 832.5 mg.

Tablets were visually inspected if lamination or capping occurred. To evaluate the capping tendency in more detail, the oval tablets were additionally crushed lengthwise using a standard hardness tester. Tablets offering a separation into layers (e.g. separation of the upper or lower cap) were declared as "capped", tablets breaking just clearly into two halfs were counted as non-capped.

Disintegration experiments were performed according to European Pharmacopoeia 9.2 (2017) section 2.9.1 Disintegration of tablets and capsules using a disk. The mean of the measurement times was calculated. The dissolution experiments were performed according to the USP 40 (chapter <711> Dissolution) using apparatus 2 (paddle method). Stirrer speed was between 50 or 75 rpm, release medium phosphate buffer pH 6.8 (1 L) and temperature 37 °C. Capping tests were performed for n=10 tablets. For disintegration 3-6 tablets were used and for dissolution experiments 6 tablets were tested.

### Example 2: Process for production of fixed dose composition tablet formulation and methods for analysis

Roll compaction/dry granulation was perfomed with an instrumented roller compactor (Minipactor 250/25, Gerteis Maschinen + Prozessengineering AG, Jona, Switzerland). A gap width of 3 mm, a roll speed of 2.5 rpm and a specific compaction force of 5 kN/cm was used. Ribbons were directly dry granulated through a 1 mm sieve with a star granulator, rotating 250° clockwise and 350° counterclockwise and the rotor speed set to 60 rpm clockwise and counterclockwise.

Granules were postblended and afterwards compressed to oval shaped tablets (18x8 mm and 19x9 mm) with a weight between 662.5 and 882.5 mg. Compression experiments where performed with an instrumented rotary die press (T 200, Kilian) and a compression simulator (Styl'One Evolution, Medelpharm). For the compression experiments different compression speeds (15, 40 and 72 rpm) as well as different compression pressures (between 15 and 45 kN) were used.

Tablets were characterized with respect to their weight, height, length, width and breaking load. To evaluate the capping tendency in more detail, the oval tablets were crushed lengthwise using a standard hardness tester. Tablets offering a separation into layers (e.g. separation of the upper or lower cap) were declared as "capped", tablets breaking just clearly into two halfs were counted as non capped.

Disintegration experiments were performed according to Ph. Eur. using a disk. All measurements beside the disintegration and dissolution were performed for n=10 tablets. For disintegration 3-6 tablets were used and for dissolution experiments 6 tablets were tested.

LoD, uniformity of mass, disintegration and friability can be and were determined as known in the art and as described in Ph. Eur.. Thickness of uncoated or coated tablets can be determined as known in the art, e.g. by manual measurement with caliper or micrometer or automatic measurement with electronic gauge. The breaking load of the tablets is determined by a three-point bending test using a standard tablet hardness tester and a pair of breaking load measurement jaws. The measurement will be performed with tablets laying on the height. For the measurement the insert with the single, central, not adjustable spike will be placed on the movable jaw of the hardness tester. The insert with the two movable spikes will be placed on the unmovable jaw of the hardness tester. The distance (span) between the movable spikes has to be adjusted (screw, scale), in such a way, that 2/3 of the length of the tablet is laying between the spikes and 1/3 outside (two equal parts right and left).

**Table 1. Tests and procedures for parameter characterization.**

| **Test** | **Procedure** |
|---|---|
| **Blend** Loss of drying | Ph. Eur. |
| **Uncoated tablets** Uniformity of mass ^{b} | Ph. Eur. |
| Thickness | manual measurement with caliper or micrometer or automatic measurement with electronic gauge |
| Breaking load (mean value) | instrumental determination of breaking load |
| Disintegration (with disk) | Ph. Eur. |
| Friability | Ph. Eur. |

### Example 3: Fixed dose formulations pursuant to the invention

Tablets made from a fixed dose combination formulation were prepared as described above but without adding water. All tablets comprised acarbose and metformin in a ratio of 1:10 at 50 mg acarbose and 500 mg metformin hydrochloride salt. All formulations comprised Aerosil as a flow agent at 4 mg/tablet and Magnesium stearate at 3.5 mg/Tablet. Avicel (CAS 9004-34-6) is a microcrystalline cellulose and HPC is an abbreviation of hydroxypropylcellulose, while the HPC L is a hydroxypropylcellulose of a viscosity of 6-10mPas, a molecular weight of about 140.000 g/mol and a particle size D50 of about 160 µm, D90 of 355 µm respectively and HPC SSL SFP is an hydroxypropylcellulose of a viscosity of 2-3 mPas, a molecular weight of about 40,000 g/mol and a particle size D50 of about 20 µm, D90 of 50 µm respectively. Polyplasdones have been described above; these are cross-linked polyvinylpyrrolidones of different grades with regard to average particle size.

**Table 2. Examples for the composition of different formulations**

| Formulation # | Avicel^{®} PH- 101 [mg] | Dry Binder [mg] | | Disintegrant [mg] | | Overall weight/tablet (w/o coating) [mg] |
|---|---|---|---|---|---|---|
| | | HPC-L | HPC-SSL-SFP | Polyplasdone^{®} XL | Polyplasdone ^{®}XL 10 | |
| 1 | 150 | 0 | 100 | 0 | 25 | 832.5 |
| 2 | 100 | 0 | 80 | 0 | 25 | 762.5 |
| 3 | 70 | 0 | 80 | 0 | 35 | 742.5 |
| 4 | 70 | 20 | 60 | 0 | 35 | 742.5 |
| 5 | 70 | 20 | 60 | 35 | 0 | 742.5 |

### Example 4: Other fixed dose formulations

Tablets made from a fixed dose combination formulation were prepared as described above but without adding water. All tablets comprised acarbose and metformin in a ratio of 1:10 at 50 mg acarbose and 500 mg metformin hydrochloride salt. All formulations comprised Aerosil as a flow agent at 4.0 mg/tablet and magnesium stearate at 3.5 mg/Tablet.

**Table 3. Eight different formulations as comparative examples. HPC is an abbreviation of hydroxypropylcellulose while L-HPC NBD 21 is also a low-substituted hydroxypropylcellulose which is chemically identical to the current L-HPC grades. METHOCEL^{™} is the trade name of cellulose ethers while Methocel E3 Premium LV is characterized by a methoxyl substitution of 28 - 30% and a hydroxypropyl substitution of 7-12%.**

| Comparative Example # | Avicel^{®} PH- 101 [mg] | Dry Binder [mg] | | | | | | Disintegrant [mg] | | Overall weight/tablet (w/o coating) [mg] |
|---|---|---|---|---|---|---|---|---|---|---|
| | | HPC-L | Kollidon^{®} VA64 | Kollidon^{®} VA64F | MethocelTM E3 Premium LV | L-HPC LH 11 | L-HPC NBD 21 | Pol yplasdone^{®} XL | Pol yplasdone^{®} XL 10 | |
| C1 | 150 | 0 | 0 | 0 | 100 | 0 | 0 | 0 | 25 | 832.5 |
| C2 | 150 | 100 | 0 | 0 | 0 | 0 | 0 | 0 | 25 | 832.5 |
| C3 | 150 | 0 | 0 | 0 | 0 | 100 | 0 | 0 | 25 | 832.5 |
| C4 | 150 | 0 | 0 | 0 | 0 | 0 | 100 | 0 | 25 | 832.5 |
| C5 | 50 | 0 | | 70 | 0 | 0 | 0 | 0 | 35 | 712.5 |
| C6 | 70 | 0 | 0 | 80 | 0 | 0 | 0 | 0 | 35 | 742.5 |
| C7 | 50 | 100 | 0 | 0 | 0 | 0 | 0 | 25 | 0 | 737.5 |
| C8 | 70 | | 80 | 0 | 0 | 0 | 0 | 0 | 35 | 742.5 |

### Example 5: Assessment of the characteristics of the tablets

Capping behavior, disintegration time and release of acarbose and metformin was determined for the formulations according to examples 3 and 4 as described above (table 3). The formulations according to table 3, which comprise a variety of alternative dry binders, collectively show capping. Favorable capping behaviour was observed for all formulations described in table 2 (see table 4). Relationship between moisture content (percentages) and capping for different compression forces for selected formulations are shown in table 5. Relationship between moisture content (MC) and capping for different breaking loads and two selected formulations are shown in table 6.

**Table 4. Capping behavior, disintegration time and release of acarbose and metformin for the formulations according to table 2 and 3.**

| Example | Capping | Disintegration time [seconds] | Release acarbose [% of labeled amount after 30 min] | Release metformin [% of labeled amount after 30 min] |
|---|---|---|---|---|
| 1 | NO | 700 | 69.3 | 84.4 |
| 2 | NO | 533 | na | na |
| 3 | NO | 628 | 87.03 | 96.7 |
| 4 | NO | 414 | na | na |
| 5 | NO | 450 | 92.9 | 94.2 |
| C1 | YES | 131 | 92 | 97.1 |
| C2 | YES | 198 | 87.3 | 93.5 |
| C3 | YES | na | na | na |
| C4 | YES | na | na | na |
| C5 | YES | 243 | na | na |
| C6 | YES | 313 | 89.4 | 95.71 |
| C7 | YES | 117 | na | na |

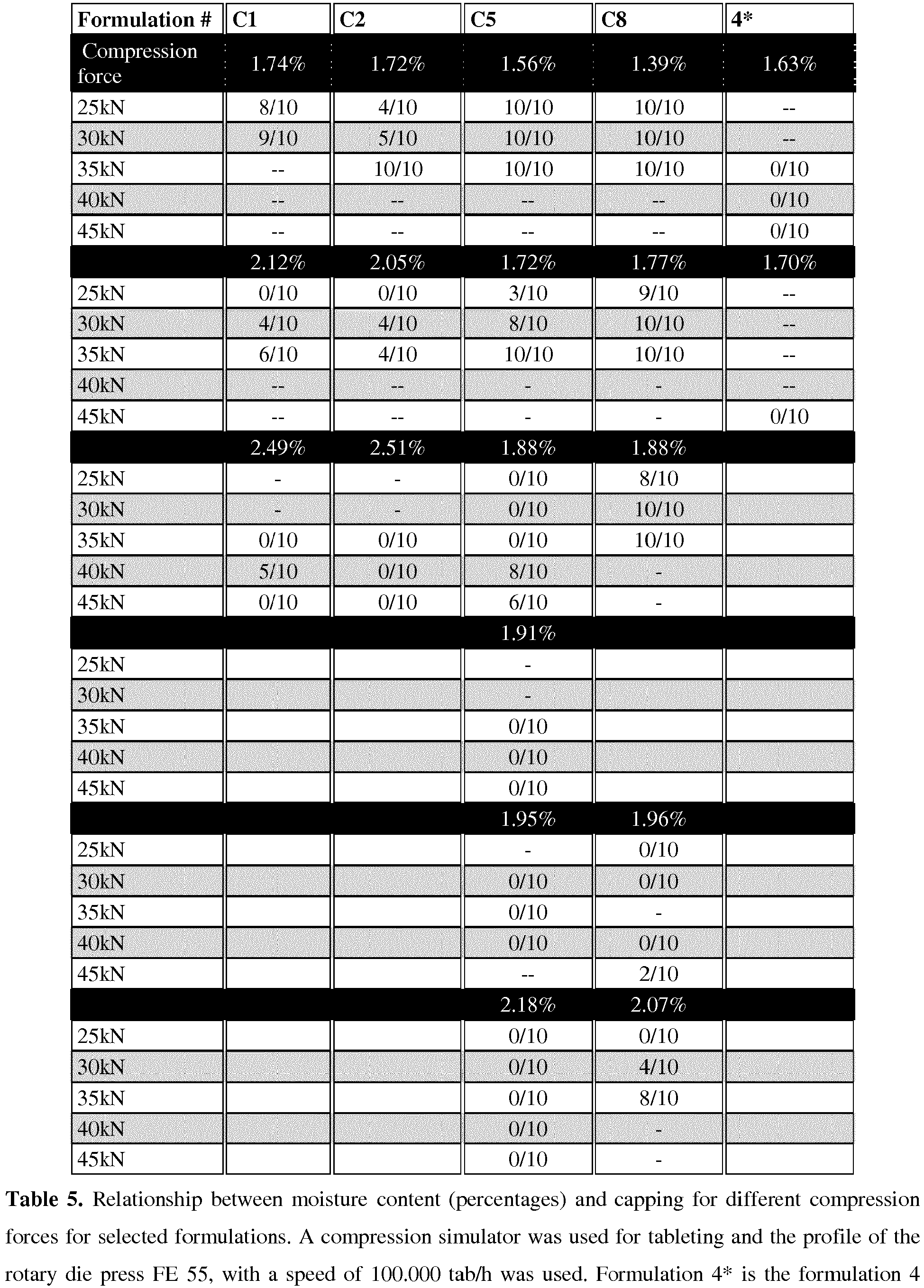

according to example 3 comprising 4 mg magnesium stearate/tablet instead of 3.5 mg magnesium stearate/tablet. Favorable capping behaviour was observed for tight control of moisture content.

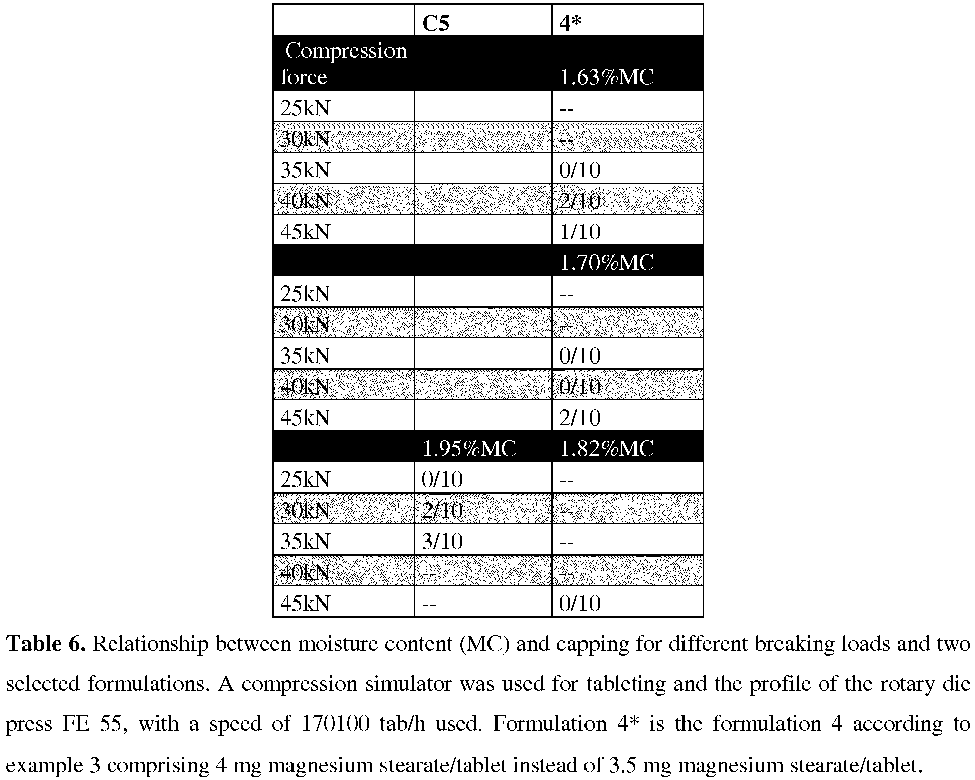

## Claims

1. Fixed dose tablet formulation comprising
metformin and acarbose,
at least one disintegrant,
at least one flow agent and/or lubricant,
**characterized in that** the moisture content of said formulation is 1.6% to 3.0%,
and a hydroxypropylcellulose as dry binder, which has a viscosity of 2 to 3 mPas at a molecular weight of about 40,000 g/mol and a particle size D50 of about 20 µm, D90 of 50 µm respectively, and
another hydroxypropylcellulose as dry binder that has a viscosity of 6 to 10 mPas at a molecular weight of about 140,000 g/mol and a particle size D50 of about 160 µm, D90 of 335 µm or 355 µm respectively.

2. Fixed dose tablet formulation according to Claim 1, wherein the ratio of the hydroxypropylcellulose with a viscosity of 2 to 3 mPas at a molecular weight of about 40,000 g/mol and a particle size D₅₀ of about 20 µm, D₉₀ of 50 µm respectively towards the hydroxypropylcellulose with a viscosity of 6 to 10 mPas at a molecular weight of about 140,000 g/mol and a particle size D₅₀ of about 160 µm, D₉₀ of 335 µm or 355 µm respectively is between 4:1 and 1:1, particularly about 3:1.

3. Fixed dose tablet formulation according to any one of the previous Claims, comprising acarbose and metformin at a weight ratio between 1 to 17 and 1 to 4 (e.g. of about 1 to 10, or about 1 to 8.5 or about 1 to 5), wherein optionally the overall amount of the actives is at least 60% by weight of said formulation.

4. Fixed dose tablet formulation according to any one of the previous Claims, wherein the dry binders are present in the formulation in an overall amount from about 5% to 15% by weight, more preferably from about 7% to 12% by weight, even more preferably from about 8% to 11% by weight.

5. Fixed dose tablet formulation according to any one of the previous Claims, wherein cross-linked polyvinylpyrrolidone is the disintegrant.

6. Fixed dose tablet formulation according to any one of the previous Claims, wherein said formulation comprises magnesium stearate as a lubricant.

7. Fixed dose tablet formulation according to any one of the previous Claims, further comprising microcrystalline cellulose as an excipient with disintegration promoting properties.

8. Fixed dose tablet formulation according to any one of the previous Claims wherein the formulation comprises
a) about 74% by weight of acarbose and metformin in a ratio of 1:10,
b) about 10.5% by weight of dry binders, consisting of about 2.5% by weight of hydroxypropylcellulose with a viscosity of 6 to10 mPas, a molecular weight of about 140,000 g/mol and a particle size D₅₀ of about 160 µm, and/or D₉₀ of 335 µm or 355 µm respectively and about 8% by weight of hydroxypropylcellulose with a viscosity of 2 to3 mPas, a molecular weight of about 40,000 g/mol and a particle size D₅₀ of about 20 µm, and/or D₉₀ of 50 µm respectively,
c) about 9% by weight of microcrystalline cellulose as an excipient with disintegration promoting properties
d) about 4.5% by weight of a disintegrant being cross-linked polyvinylpyrrolidone with an average particle size of about 30µm, and
e) about 0.5% by weight of both a lubricant and a flow agent, wherein the flow agent is highly-disperse silica and the lubricant is magnesium stearate.

9. A tablet comprising a fixed dose tablet formulation according to any one of the previous Claims.

10. A tablet according to Claim 9, wherein said tablet has a length of about 18 mm, a width of about 8 mm and a thickness of about 6.3 to 7 mm.

11. A coated tablet comprising a core made up from a tablet according to any one of claims 9 to 10 **characterized in that** such core is coated.

12. A coated tablet according to Claim 11, wherein the coating is a mixture of about 60 wt.% hydroxypropylmethylcellulose, about 16 wt.% titanium dioxide, about 4 wt.% ferric oxide yellow and about 20 wt.% polyethylene glycol.

13. A process for the production of a fixed dose tablet formulation according to any of Claims 1 to 8 comprising the steps of
a) dry mixing metformin and acarbose, e.g. together with at least one dry binder and at least one disintegrant,
b) dry granulating the resulting mixture to obtain dry granules,
c) optionally at least once blending said dry granules with a further excipient, and
d) optionally arranging the fixed dose combination formulation in the form of a tablet (tableting).

14. A process for the production of a tablet according to any one of Claims 9 to 10, wherein said process comprises the steps of
a) dry mixing metformin and acarbose, e.g. together with at least one dry binder and at least one disintegrant,
b) dry granulating the resulting mixture to obtain dry granules, preferably by roller compaction with a throughput of at least 20 kg/h,
c) at least once blending said dry granules with at least one flow agent and/or lubricant, and
d) tableting said fixed dose combination formulation of acarbose and metformin.

15. A process according to any of Claims 13 or 14 further comprising the steps of
a) determining the moisture content of the formulation and/or the dry granules and
b) adding water if the formulation moisture content as determined in step a) is below 1.6%.

16. A process for the production of a coated tablet according to Claims 11 or 12, wherein said process comprises a process according to any of Claims 13 to 15 and further comprises another step of coating the tablet.

## Patentansprüche

1. Fixdosis-Tablettenformulierung, umfassend Metformin und Acarbose,
mindestens ein Zerfallsmittel,
mindestens ein Fließmittel und/oder Schmiermittel, **dadurch gekennzeichnet, dass** der Feuchtigkeitsgehalt der Formulierung 1,6 % bis 3,0 % beträgt,
und eine Hydroxypropylcellulose als trockenes Bindemittel mit einer Viskosität von 2 bis 3 mPas bei einem Molekulargewicht von etwa 40.000 g/mol und einer Teilchengröße D50 von etwa 20 µm, bzw. D90 von 50 µm und
eine weitere Hydroxypropylcellulose als trockenes Bindemittel mit einer Viskosität von 6 bis 10 mPas bei einem Molekulargewicht von etwa 140.000 g/mol und einer Teilchengröße D50 von etwa 160 µm, bzw. D90 von 335 µm oder 355 µm.

2. Fixdosis-Tablettenformulierung nach Anspruch 1, wobei das Verhältnis von Hydroxypropylcellulose mit einer Viskosität von 2 bis 3 mPas bei einem Molekulargewicht von etwa 40.000 g/mol und einer Teilchengröße D₅₀ von etwa 20 µm, bzw. D₉₀ von 50 µm zu Hydroxypropylcellulose mit einer Viskosität von 6 bis 10 mPas bei einem Molekulargewicht von etwa 140.000 g/mol und einer Teilchengröße D₅₀ von etwa 160 µm, bzw. D₉₀ von 335 µm oder 355 µm zwischen 4:1 und 1:1 ist, insbesondere etwa 3:1.

3. Fixdosis-Tablettenformulierung nach einem der vorhergehenden Ansprüche, umfassend Acarbose und Metformin in einem Gewichtsverhältnis zwischen 1 zu 17 und 1 zu 4 (z. B. etwa 1 zu 10 oder etwa 1 zu 8,5 oder etwa 1 zu 5), wobei optional die Gesamtmenge der Wirkstoffe mindestens 60 Gew.-% der Formulierung beträgt.

4. Fixdosis-Tablettenformulierung nach einem der vorhergehenden Ansprüche, wobei die trockenen Bindemittel in der Formulierung in einer Gesamtmenge von etwa 5 bis 15 Gew.-%, vorzugsweise von etwa 7 bis 12 Gew.-%, noch weiter bevorzugt von etwa 8 bis 11 Gew.-%, vorliegen.

5. Fixdosis-Tablettenformulierung nach einem der vorhergehenden Ansprüche, wobei es sich bei dem Zerfallsmittel um vernetztes Polyvinylpyrrolidon handelt.

6. Fixdosis-Tablettenformulierung nach einem der vorhergehenden Ansprüche, wobei die Formulierung als Schmiermittel Magnesiumstearat umfasst.

7. Fixdosis-Tablettenformulierung nach einem der vorhergehenden Ansprüche, ferner umfassend mikrokristalline Cellulose als Hilfsstoff mit zerfallsfördernden Eigenschaften.

8. Fixdosis-Tablettenformulierung nach einem der vorhergehenden Ansprüche, wobei die Formulierung
a) etwa 74 Gew.-% Acarbose und Metformin in einem Verhältnis von 1:10,
b) etwa 10,5 Gew.-% trockene Bindemittel, bestehend aus etwa 2,5 Gew.-% Hydroxypropylcellulose mit einer Viskosität von 6 bis 10 mPas, einem Molekulargewicht von etwa 140.000 g/mol und einer Teilchengröße D₅₀ von etwa 160 µm und/oder D₉₀ von 335 µm oder 355 µm respektive und etwa 8 Gew.-% Hydroxypropylcellulose mit einer Viskosität von 2 bis 3 mPas, einem Molekulargewicht von etwa 40.000 g/mol und einer Teilchengröße D₅₀ von etwa 20 µm und/oder D₉₀ von 50 µm respektive,
c) etwa 9 Gew.-% mikrokristalline Cellulose als Hilfsstoff mit zerfallsfördernden Eigenschaften,
d) etwa 4,5 Gew.-% eines Zerfallsmittels, bei dem es sich um vernetztes Polyvinylpyrrolidon mit einer durchschnittlichen Teilchengröße von etwa 30 µm handelt, und
e) etwa 0,5 Gew.-% sowohl eines Schmiermittels als auch eines Fließmittels, wobei es sich bei dem Fließmittel um hochdisperse Silica handelt und es sich bei dem Schmiermittel um Magnesiumstearat handelt,
umfasst.

9. Tablette, umfassend eine Fixdosis-Tablettenformulierung nach einem der vorhergehenden Ansprüche.

10. Tablette nach Anspruch 9, wobei die Tablette eine Länge von etwa 18 mm, eine Breite von etwa 8 mm und eine Dicke von etwa 6,3 bis 7 mm aufweist.

11. Überzogene Tablette, umfassend einen Kern, der aus einer Tablette nach einem der Ansprüche 9 bis 10 besteht, **dadurch gekennzeichnet, dass** der Kern überzogen ist.

12. Überzogene Tablette nach Anspruch 11, wobei es sich bei dem Überzug um eine Mischung von etwa 60 Gew.-% Hydroxypropylmethylcellulose, etwa 16 Gew.-% Titandioxid, etwa 4 Gew.-% Eisenoxidgelb und etwa 20 Gew.-% Polyethylenglykol handelt.

13. Verfahren zur Herstellung einer Fixdosis-Tablettenformulierung nach einem der Ansprüche 1 bis 8, das folgende Schritte umfasst:
a) Trockenmischen von Metformin und Acarbose, z. B. zusammen mit mindestens einem trockenen Bindemittel und mindestens einem Zerfallsmittel,
b) Trockengranulieren der resultierenden Mischung zum Erhalt von trockenem Granulat,
c) gegebenenfalls mindestens einmaliges Mischen des trockenen Granulats mit einem weiteren Hilfsstoff und
d) optional Herrichten der Fixdosis-Kombinationsformulierung in Form einer Tablette (Tablettieren).

14. Verfahren zur Herstellung einer Tablette nach einem der Ansprüche 9 bis 10, wobei das Verfahren folgende Schritte umfasst:
a) Trockenmischen von Metformin und Acarbose, z. B. zusammen mit mindestens einem trockenen Bindemittel und mindestens einem Zerfallsmittel,
b) Trockengranulieren der resultierenden Mischung zum Erhalt von trockenem Granulat, vorzugsweise durch Walzenkompaktierung mit einem Durchsatz von mindestens 20 kg/h,
c) mindestens einmaliges Mischen des trockenen Granulats mit einem Fließmittel und/oder Gleitmittel und
d) Tablettieren der Fixdosis-Kombinationsformulierung von Acarbose und Metformin.

15. Verfahren nach Anspruch 13 oder 14, das ferner folgende Schritte umfasst:
a) Bestimmen des Feuchtigkeitsgehalts der Formulierung und/oder des trockenen Granulats und
b) Zugeben von Wasser, wenn der wie in Schritt a) bestimmte Feuchtigkeitsgehalt der Formulierung unter 1,6 % liegt.

16. Verfahren zur Herstellung einer überzogenen Tablette nach Anspruch 11 oder 12, wobei das Verfahren ein Verfahren nach einem der Ansprüche 13 bis 15 umfasst und ferner einen weiteren Schritt des Überziehens der Tablette umfasst.

## Revendications

1. Formulation de comprimé à dose fixe comprenant
de la metformine et de l'acarbose,
au moins un désintégrant,
au moins un agent d'écoulement et/ou lubrifiant, **caractérisée en ce que** la teneur en humidité de ladite formulation est de 1,6 % à 3,0 %,
et une hydroxypropylcellulose en tant que liant sec, qui possède une viscosité de 2 à 3 mPas à un poids moléculaire d'environ 40 000 g/mole et une taille de particule D50 d'environ 20 µm, D90 de 50 µm respectivement, et
une autre hydroxypropylcellulose en tant que liant sec, qui possède une viscosité de 6 à 10 mPas à un poids moléculaire d'environ 140 000 g/mole et une taille de particule D50 d'environ 160 µm, D90 de 335 µm ou 355 µm respectivement.

2. Formulation de comprimé à dose fixe selon la revendication 1, le rapport de l'hydroxypropylcellulose dotée d'une viscosité de 2 à 3 mPas à un poids moléculaire d'environ 40 000 g/mole et d'une taille de particule D₅₀ d'environ 20 µm, D₉₀ de 50 µm respectivement sur l'hydroxypropylcellulose dotée d'une viscosité de 6 à 10 mPas à un poids moléculaire d'environ 140 000 g/mole et d'une taille de particule D₅₀ d'environ 160 µm, D₉₀ de 335 µm ou 355 µm respectivement étant compris entre 4 : 1 et 1 : 1, particulièrement étant d'environ 3 : 1.

3. Formulation de comprimé à dose fixe selon l'une quelconque des revendications précédentes, comprenant de l'acarbose et de la metformine à raison d'un rapport en poids compris entre 1 sur 17 et 1 sur 4 (par ex. d'environ 1 sur 10, ou d'environ 1 sur 8,5 ou d'environ 1 sur 5), éventuellement la quantité globale des substances actives étant d'au moins 60 % en poids de ladite formulation.

4. Formulation de comprimé à dose fixe selon l'une quelconque des revendications précédentes, les liants secs étant présent dans la formulation en une quantité globale d'environ 5 % à 15 % en poids, plus préférablement d'environ 7 % à 12 % en poids, encore plus préférablement d'environ 8 % à 11 % en poids.

5. Formulation de comprimé à dose fixe selon l'une quelconque des revendications précédentes, une polyvinylpyrrolidone réticulée étant le désintégrant.

6. Formulation de comprimé à dose fixe selon l'une quelconque des revendications précédentes, ladite formulation comprenant du stéarate de magnésium en tant que lubrifiant.

7. Formulation de comprimé à dose fixe selon l'une quelconque des revendications précédentes, comprenant en outre une cellulose microcristalline en tant qu'excipient doté de propriétés favorisant la désintégration.

8. Formulation de comprimé à dose fixe selon l'une quelconque des revendications précédentes, la formulation comprenant
a) environ 74 % en poids d'acarbose et de metformine en un rapport de 1 : 10,
b) environ 10,5 % en poids de liants secs, constitués d'environ 2,5 % en poids d'hydroxypropylcellulose dotée d'une viscosité de 6 à 10 mPas, d'un poids moléculaire d'environ 140 000 g/mole et d'une taille de particule D₅₀ d'environ 160 µm, et/ou D₉₀ de 335 µm ou 355 µm respectivement et d'environ 8 % en poids d'hydroxypropylcellulose dotée d'une viscosité de 2 à 3 mPas, d'un poids moléculaire d'environ 40 000 g/mole et d'une taille de particule D₅₀ d'environ 20 µm, et/ou D₉₀ de 50 µm respectivement,
c) environ 9 % en poids de cellulose microcristalline en tant qu'excipient doté de propriétés favorisant la désintégration
d) environ 4,5 % en poids d'un désintégrant qui est une polyvinylpyrrolidone réticulée dotée d'une taille moyenne de particule d'environ 30 µm, et
e) environ 0,5 % en poids à la fois d'un lubrifiant et d'un agent d'écoulement, l'agent d'écoulement étant une silice hautement dispersée et le lubrifiant étant du stéarate de magnésium.

9. Comprimé comprenant une formulation de comprimé à dose fixe selon l'une quelconque des revendications précédentes.

10. Comprimé selon la revendication 9, ledit comprimé possédant une longueur d'environ 18 mm, une largeur d'environ 8 mm et une épaisseur d'environ 6,3 à 7 mm.

11. Comprimé revêtu comprenant un noyau composé d'un comprimé selon l'une quelconque des revendications 9 et 10 **caractérisé en ce qu'**un tel noyau est revêtu.

12. Comprimé revêtu selon la revendication 11, le revêtement étant un mélange d'environ 60 % en poids d'hydroxypropylméthylcellulose, d'environ 16 % en poids de dioxyde de titane, d'environ 4 % en poids d'oxyde de ferrique jaune et d'environ 20 % en poids de polyéthylène glycol.

13. Procédé pour la production d'une formulation de comprimé à dose fixe selon l'une quelconque des revendications 1 à 8 comprenant les étapes de
a) mélange à sec de metformine et d'acarbose, par ex. conjointement avec au moins un liant sec et au moins un désintégrant,
b) granulation à sec du mélange résultant pour obtenir des granulés secs,
c) éventuellement assemblage au moins une fois desdits granulés secs avec un excipient supplémentaire, et
d) éventuellement mise en forme de la formulation de combinaison à dose fixe sous la forme d'un comprimé (mise en comprimé).

14. Procédé pour la production d'un comprimé selon l'une quelconque des revendications 9 et 10, ledit procédé comprenant les étapes de
a) mélange à sec de metformine et d'acarbose, par ex. conjointement avec au moins un liant sec et au moins un désintégrant,
b) granulation à sec du mélange résultant pour obtenir des granulés secs, préférablement par compactage au rouleau avec un débit d'au moins 20 kg/h,
c) assemblage au moins une fois desdits granulés secs avec au moins un agent d'écoulement et/ou lubrifiant, et
d) mise en comprimé de ladite formulation de combinaison à dose fixe d'acarbose et de metformine.

15. Procédé selon l'une quelconque des revendications 13 et 14 comprenant en outre les étapes de
a) détermination de la teneur en humidité de la formulation et/ou des granulés secs et
b) ajout d'eau si la teneur en humidité de la formulation, telle que déterminée dans l'étape a), est inférieure à 1,6 %.

16. Procédé pour la production d'un comprimé revêtu selon la revendication 11 ou 12, ledit procédé comprenant un procédé selon l'une quelconque des revendications 13 à 15 et comprenant en outre une autre étape de revêtement du comprimé.
